# EUROPEAN PATENT APPLICATION

(11) **EP 4 043 585 A1**
(43) Date of publication of application: **17.08.2022**
(21) Application number: 20873439.2
(22) Date of filing: 29.09.2020
(51) Int. Cl.: C12Q 1/6881

(54) **METHOD FOR DETERMINING IF ORIGIN OF BIOLOGICAL SAMPLE IS FROM LIVER TISSUE**

(30) Priority: 08.10.2019 KR 20190124549
(71) Applicant: Lepidyne Co., Ltd., Seongdong-gu Seoul 04779 (KR)
(72) Inventor: KIM, Young Joon, Seoul 06024 (KR); KIM, Da Won, Seoul 06366 (KR); HA, Jeong Sil, Seoul 06024 (KR)
(74) Representative: Grünecker Patent- und Rechtsanwälte PartG mbB
(86) International application number: PCT/KR2020/013290
(87) International publication number: WO 2021/071164

(57) **Abstract**

The present invention relates to a method for determining whether a biological sample of unknown origin is derived from liver tissue and a composition comprising a liver tissue-specific DNA methylation marker for performing the same, and the liver tissue-specific DNA methylation marker has a low methylation level in other tissue except for liver tissue and has a high methylation level in normal liver tissue and liver cancer tissue, and thus, it can determine whether a biological sample is derived from liver tissue with excellent accuracy.

<110>    LEPIDYNE CO., LTD.

<120>    Method for Identifying Whether Biological Sample is Derived from
         Liver Tissue

<130>    X20U16C0163

<150>    KR 10-2019-0124549
<151>    2019-10-08

<160>    2

<170>    KoPatentIn 3.0

<210>    1
<211>    122
<212>    DNA
<213>    Artificial Sequence

<220>
<223>    polynucleotides


<400>    1
ggggacacga ctgccccagc aacttgcagg agtcgcacca cctccatgca cttgtcccgg        60

cgctcccggc ccgagtagcc tcccgcagcc cacacctgcc ctggcagttc gcaccctagc       120

ag                                                                      122


<210>    2
<211>    122
<212>    DNA
<213>    Artificial Sequence

<220>
<223>    polynucleotides


<400>    2
ttcccattgg ttgagacagc accgcccagc caaagccccc ttgtcctcgc gcgggtgcgc        60

cgcctggact cccaccctgg ccagtcccgg gcccaccacc actctggcat ccccagcctg       120

tc                                                                      122

## Description

### [TECHNICAL FIELD]

The present invention relates to a method for determining whether a biological sample of unknown origin is derived from liver tissue, and a composition comprising a liver tissue-specific DNA methylation marker for performing the same.

### [BACKGROUND ART]

Cells in the human body have the same genetic information, but the functions and shapes of each cell are very diverse. This is because specific genes are expressed in each cell, and accordingly, the cell differentiation process is different, resulting in a difference in cell phenotype. Several factors, such as DNA methylation, histone modification, tissue-specific transcription factors are involved in expression of these specific genes. In particular, DNA methylation, specifically, methylation of the CpG site, is an essential element for cell-specific gene expression and is known to have unique DNA methylation characteristics for each cell or tissue. Therefore, the origin of a cell or tissue can be easily identified by using DNA methylation characteristics.

Identification of the origin of a cell or tissue can enable early diagnosis of diseases and increase the accuracy of diagnosis. For example, circulating cell free DNA (cfDNA) is present in blood circulating in a living body, and among them, there may be circulating tumor DNA (ctDNA) flowed out from tumor cells. Even if the presence of ctDNA is detected by a liquid biopsy, in order to accurately diagnose cancer, it is necessary to additionally confirm the tissue from which the ctDNA originated, and in this process, diagnosis of cancer is delayed. However, early diagnosis of cancer is possible if the tissue from which it originated can be identified while detecting the corresponding ctDNA, and a method for identifying the origin of a biological sample is increasingly needed for early diagnosis of other diseases as well as cancer.

The present inventors have studied a method for identifying the origin of a tissue and cell of unknown origin based on DNA methylation data, and as a result, have confirmed a marker with a high methylation level specifically for liver tissue, thereby completing the present invention.

### [DISCLOSURE]

### [TECHNICAL PROBLEM]

An object of the present invention is to provide a method for determining whether a biological sample of unknown origin is derived from liver tissue and a composition for performing the method.

### [TECHNICAL SOLUTION]

In order to achieve the above object, one aspect of the present invention provides a method for determining whether a biological sample originates from liver tissue comprising the following steps:
(a) separating DNA from an isolated biological sample of a subject; and
(b) measuring methylation levels of CpG site of the sequences represented by SEQ ID NO: 1 and SEQ ID NO: 2 in the separated DNA.

In one specific embodiment of the present invention, the subject may be a human, and the biological sample comprises tissue, tissue fragments, cells, cell fragments, blood, plasma, body fluids, feces and urine isolated from the subject, and the like, but not limited thereto. The tissue, tissue fragments, cells and cell fragments, and the like may be separated from the blood, plasma, body fluids, urine and the like collected from the subject. In addition, the DNA may be DNA isolated from tissue, cells, and the like, and may be cell free DNA (cfDNA) floating in the blood, plasma, body fluids and the like or circulating tumor DNA (ctDNA) flowed out of tumor cells.

The term used in the present specification, "methylation" means attachment of a methyl group (-CH₃) to a base constituting DNA, and preferably, means methylation occurring at a cytosine of a specific CpG site in specific DNA. The term, "methylation level" refers to quantitative evaluation of methylation status of CpG site present in specific DNA sequence, and the methylation status refers to the presence or absence of 5-methyl-cytosine at one or more CpG sites in the DNA sequence.

The term used in the present specification, "CpG site" refers to a sequence in which cytosine (C) and guanine (G) are linked by a phosphate group, and it may be present in a DNA sequence comprising a promoter region, a protein coding region (open reading frame, ORF) and a terminator region, and the like. The methylation of CpG site is known to involve in maintaining genome stability and regulating gene expression, and the like.

The present inventors have tried to discover a liver tissue-specific methylation marker, and as a result, have discovered cg12137206 (SEQ ID NO: 1) and cg03792768(SEQ ID NO: 2) markers which has high methylation level in normal liver tissue and liver cancer tissue samples and has low methylation level in other tissues including blood. The target methylation sites of the two markers are CpG sites positioned in the 61st nucleotide in the sequence represented by SEQ ID NO: 1 and SEQ ID NO: 2, respectively. However, as methylation may occur even in other CpG sites in addition to the target methylation sites, in the present invention, the methylation level of the total CpG sites present in the sequences represented by SEQ ID NO: 1 and 2 comprising the target CpG sites can be measured.

In one specific embodiment of the present invention, the (b) may be performed by a method selected from the group consisting of PCR, methylation specific PCR, real time methylation specific PCR, MethyLight PCR, MehtyLight digital PCR, EpiTYPER, PCR using methylated DNA specific binding protein, quantitative PCR, DNA chip, molecular beacon, MS-HRM (Methylation-sensitive high resolution melting), asymmetric PCR, asymmetric PCR MS-HRMA (asymmetric PCR Methylation-sensitive high resolution melting analysis), Recombinase Polymerase Amplification, LAMP (Loop-Mediated Isothermal Amplification), Eclipse probe, next generation sequencing panel (NGS panel), pyrosequencing and bisulfide sequencing.

For example, the methylation level may be identified by microarray, and the microarray may use a probe fixed on a solid surface. The probe may comprise a sequence complementary to 10 to 100 continuous nucleotide sequences comprising the CpG site.

In one specific embodiment of the present invention, the method may further comprise (c) comparing the methylation level to a methylation level of a normal control group, after the (b). For example, when the methylation level of the biological sample is higher compared to the methylation level of the normal control group, it can be determined that the biological sample is derived from liver tissue, and when the methylation level is lower or similar, it can be determined that the biological sample is derived from other tissue except for liver.

Another aspect of the present invention provides a method for detecting liver tissue-derived DNA in a biological sample comprising:
(a) separating DNA from an isolated biological sample of a subject; and
(b) measuring a methylation levels of CpG site of the sequences represented by SEQ ID NO: 1 and SEQ ID NO: 2 in the separated DNA.

Since the method uses a technology for measuring the methylation level of the same sequence as the method for determining whether a biological sample originates from liver tissue, contents overlapping between the two methods are omitted to avoid excessive description of the specification.

In the present invention, the method for detecting liver tissue-derived DNA may be used in combination with a conventional liver cancer diagnosis method.

Other aspect of the present invention provides a composition for determining whether a biological sample originates from liver tissue comprising agents capable of measuring methylation levels of the sequences represented by SEQ ID NO: 1 and SEQ ID NO: 2.

In one specific embodiment of the present invention, the CpG site may be 1 to a plurality comprising CpG sites positioned at the 61st nucleotide in the sequences represented by SEQ ID NO: 1 and SEQ ID NO: 2.

In one specific embodiment of the present invention, the agents capable of measuring the methylation levels may be primers, probes or antisense nucleic acids binding to the CpG sites of the sequences represented by SEQ ID NO: 1 and SEQ ID NO: 2, and the primers, probes or antisense nucleic acids may be used as a hybridizable array element and may be fixed on a substrate.

In addition, the sequence represented by SEQ ID NO: 1 or 2 may be genome DNA, and may be a sequence in which non-methylated cytosine is converted into uracil as bisulfite is treated.

The substrate is an appropriate solid or semi-solid supporter, and for example, it may comprise a film, a filter, a chip, a slide, a wafer, a fiber, a magnetic bead or non-magnetic bead, gel, tubing, a plate, a polymer, a microparticle and a capillary tube. The hybridizable array element may be fixed on the substrate by a chemical binding method, a covalent binding method such as UV or a linker (e.g.: ethylene glycol oligomer and diamine).

In one specific embodiment of the present invention, the DNA isolated from a biological sample (sample DNA) may be hybridized with the array element as being applied to hybridizable array, and the hybridization condition may be variously modified, and the detection and analysis of the hybridization level may be variously conducted according to the technology known in the art. In addition, in order to provide a signal allowing to confirm hybridization, the sample DNA and/or primer, probe or antisense nucleic acid may be labelled, and linked to oligonucleotide.

The label may comprise fluorophores (for example, fluorescein, phycoerythrin, rhodamine, lissamine, Cy3 and Cy5 (Pharmacia)), chromophores, chemical luminophores, magnetic particles, radioactive isotopes (P32 and S35), enzymes (alkaline phosphatase or horseradish peroxidase), cofactors, substrates for enzymes, heavy metals (for example, gold), antibodies, streptavidin, biotin, digoxigenin and haptene having a specific binding partner such as a chelating group. but not limited thereto.

In the present invention, the hybridization of the primer, probe or antisense nucleic acid and sample DNA depends on various factors such as reaction temperature, hybridization and washing time, buffer components and their pH and ion strength, length of the nucleotide, nucleotide sequence, amount of GC sequence, and the like. The detailed condition for the hybridization may refer to Joseph Sambrook, et al., MolecularCloning, A LaboratoryManual, Cold Spring Harbor Laboratory Press, Cold Spring Harbor, N.Y.(2001); and M.L.M. Anderson, NucleicAcid Hybridization, Springer-Verlag New York Inc. N.Y.(1999).

After the hybridization reaction, a hybridization signal generated though the hybridization reaction may be detected. For example, when the probe is labelled by enzyme, the hybridization may be confirmed by reacting the substrate of enzyme with the hybridization reaction product.

As the enzyme and substrate, peroxidase (for example, horseradish peroxidase) and chloronaphtol, aminoethylcarbozole, diaminobenzidine, D-luciferin, lucigenin (bis-N-methylacridinium nitrate), resorufin benzyl ether, luminol, Amplex red reagent (10-acetyl-3,7-dihydroxyphenoxazine), HYR (p-phenylenediamine-HCl and pyrocatechol), TMB (tetramethylbenzidine), ABTS (2,2'-Azine-di[3-ethylbenzthiazoline sulfonate]), o-phenylene diamine (OPD) and naphtol/pyronine; alkaline phosphatase and bromochloroindolyl phosphate (BCIP), nitroblue tetrazolium (NBT), naphthol-AS-B1-phosphate and ECF substrate; glucose oxidase and t-NBT (nitroblue tetrazolium) and m-PMS (phenzaine methosulfate) may be used.

In the present invention, liver tissue-specific markers have been discovered based on DNA methylation. Specifically, by progressing methylation analysis of various cell types in blood and various organ samples, markers with low methylation in other organs except for liver tissue and high methylation specifically to liver tissue were selected, and the efficiency of the markers was confirmed by machine learning. The selected markers had a high methylation level in all samples from an independent cohort normal liver tissue to liver cancer, and low methylation in other tissues including blood, and it was verified that only two markers had liver specificity with over 99% or more accuracy. A liver-specific high tendency was confirmed also in the gene expression of the corresponding markers. It has been identified that the liver-specific methylation markers can be used as major markers to confirm the origin of tissue, and it is intended to be used as an index to improve the accuracy of monitoring and early detection of liver-related diseases in the future.

### [ADVANTAGEOUS EFFECTS]

The method for determining whether a biological sample is originated from liver tissue and composition for performing the same use a liver tissue-specific DNA methylation marker, and the liver tissue-specific marker has a low methylation level in other tissue except for the liver tissue and has a high methylation level in normal liver tissue and liver cancer tissue, and therefore, whether the biological sample is derived from liver tissue can be determined with excellent accuracy.

### [BRIEF DESCRIPTION OF THE DRAWINGS]

FIG. 1 schematically shows the process of discovering a liver tissue-specific marker.
FIG. 2 shows a variable importance plot (VarImp Plot) of the discovered liver tissue-specific marker.
FIG. 3 shows the result of confirming the performance depending on the number of the discovered liver tissue-specific marker.

In FIG. 4, A shows the result of confirming the methylation level of the finally selected cg12137206 and cg03792768 markers in the normal liver tissue (Liver N) and liver cancer tissue (Liver T), and B shows the result of confirming it in the normal liver tissue (LIHC_N) and liver cancer tissue (LIHC_T), normal tissue other than liver (Pan N) and cancer tissue other than liver (Pan_T).

FIG. 5 shows the result of confirming the methylation level of cg12137206 marker in various normal tissues (A) and cancer tissues (B).

FIG. 6 shows the result of confirming the methylation level of cg03792768 marker in various normal tissues (A) and cancer tissues (B).

FIG. 7 shows the result of confirming the methylation level of cg12137206 and cg03792768 markers in major normal tissues and cancer tissues: bladder (BL), breast (BR), cervix (CE), colon (CO), esophagus (ES), glioblastoma (GB), head and neck (HN), kidney (KI), liver (LI), lung (LU), pancreas (PA), paraganglioma (PC), prostate (PR), rectum (RE), sarcoma (SA), skin (SK), stomach (ST), thymus (TH), uterine (UC) and blood (B).

### [MODE FOR INVENTION]

Hereinafter, the present invention will be described in more detail by one or more specific examples. However, these examples are intended to illustrate one or more specific examples, but the scope of the present invention is not limited by these examples.

### Example 1: Discovering of liver tissue-specific methylation markers

DNA methylation data for various carcinomas and normal tissues were downloaded from The Cancer Genome Atlas (hereinafter, referred to as TCGA). From the data downloaded from TCGA, methylated CpG sites were selected from liver cancer tissue samples (n=379), and non-methylated CpG sites were additionally selected from carcinoma samples of other tissues (n=7,260) among them. The criterion was to classify as methylated in case of 50% or more of samples were methylated, and unmethylated in case of 90% or more of samples were unmethylated.

Then, the non-methylated CpG sites in 90% of carcinoma samples (pan-tumor) other than liver cancer, and non-methylated CpG sites in 90% of normal tissue (pan-normal) other than liver were further selected.

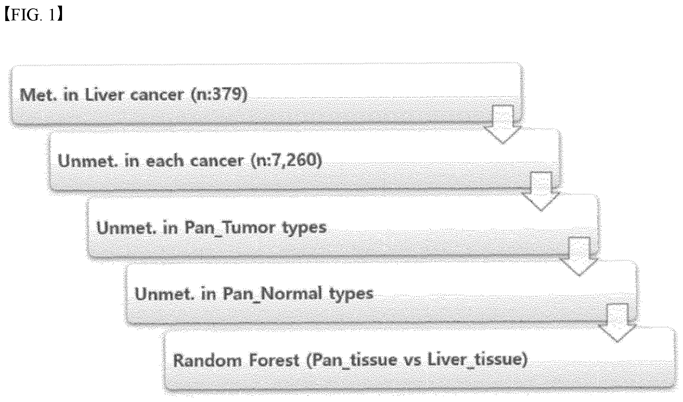

In FIG. 1, the process of discovering the liver tissue-specific markers of the present invention was schematically shown.

### Example 2: Validation of performance of liver tissue-specific methylation markers

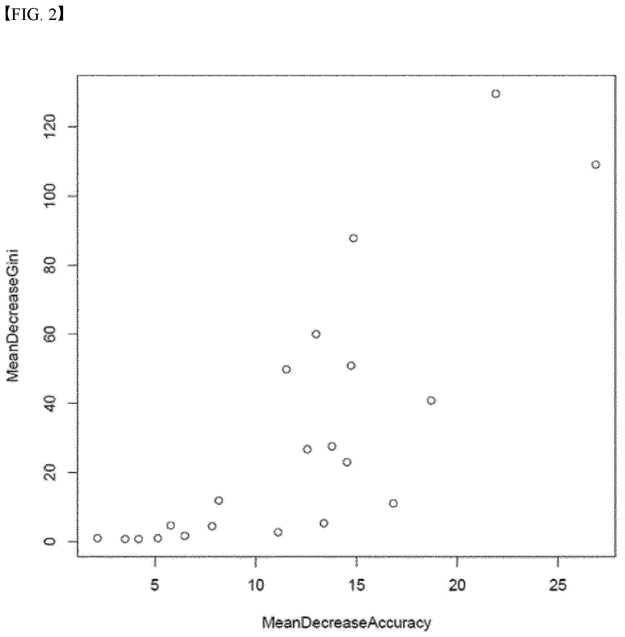

A variable importance plot (VarImp Plot) of the liver tissue-specific markers discovered in Example 1 above was prepared by the random forest method, and this was shown in FIG. 2. In FIG. 2, MeanDecreaseAccuracy of the X-axis indicates the extent to which each marker contributes to improvement of accuracy in the classification of liver tissue/other tissue, and MeanDecreaseGini of the Y-axis indicates the extent to which each marker contributes to improvement of impurity in the classification of liver tissue/other tissue. In other words, a larger value means that the marker can clearly distinguish liver tissue from other tissues.

Then, liver tissue-specific markers were tested by randomly dividing the methylation data of cancer tissue used in Example 1 into training data and validation data. As a result, as shown in Table 1 below, it could be confirmed that the discovered markers distinguished liver tissue and other tissues with high efficiency and accuracy. The performance of the liver tissue-specific markers was shown to have accuracy of 0.9988, sensitivity of 0.9884, specificity of 0.9994 and area under the curve (AUC) of 0.9999.

**[Table 1]**

| | **Type** | **Other tissue** | **Liver tissue** |
|---|---|---|---|
| **Training data** | Other tissue | 6345 | 3 |
| | Liver tissue | 7 | 337 |
| **Validation data** | Other tissue | 1586 | 1 |
| | Liver tissue | 1 | 85 |

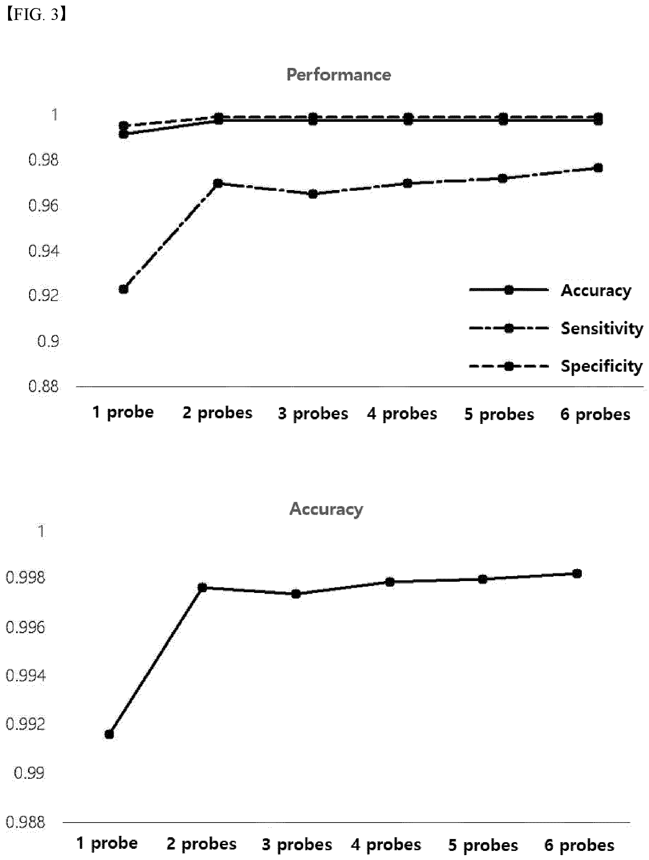

In addition, in order to confirm the performance depending on the number of markers, the test was conducted 5 times with different number of markers, and the average value was confirmed. As a result, as shown in Table 2 and FIG. 3, it was found that the performance converged to 0.999 when two or more markers are used.

**[Table 2]**

| | **1 marker** | **2 markers** | **3 markers** | **4 markers** | **5 markers** | **6 markers** |
|---|---|---|---|---|---|---|
| Accuracy | 0.9916 | 0.9976 | 0.99736 | 0.99784 | 0.99796 | 0.9982 |
| Kappa | 0.9142 | 0.97526 | 0.97264 | 0.97762 | 0.97886 | 0.98142 |
| Sensitivity | 0.923036 | 0.969686 | 0.965034 | 0.96963 | 0.971954 | 0.976718 |
| Specificity | 0.995338 | 0.999118 | 0.999118 | 0.99937 | 0.99937 | 0.999362 |
| Pos Pred Value | 0.915048 | 0.983764 | 0.983764 | 0.988172 | 0.988318 | 0.988428 |
| Neg Pred Value | 0.995844 | 0.998364 | 0.998112 | 0.998364 | 0.99849 | 0.998742 |
| Balanced Accuracy | 0.959188 | 0.984402 | 0.982078 | 0.9845 | 0.985664 | 0.988048 |

In addition, the performance of the two markers (cg12137206, cg03792768) that were found to be of high importance in the variable importance plot was further verified. As a result, as shown in Table 3 below, it could be confirmed that the markers clearly distinguished liver tissue from other tissue. It was shown to have accuracy of 0.9982, sensitivity of 0.9647, specificity of 1, and area under the curve (AUC) of 0.9939.

**[Table 3]**

| | **Type** | **Other tissue** | **Liver tissue** |
|---|---|---|---|
| **Training data** | Other tissue | 6340 | 8 |
| | Liver tissue | 11 | 333 |
| **Validation data** | Other tissue | 1587 | 3 |
| | Liver tissue | 0 | 82 |

Based on the above results, cg12137206 cg03792768 markers were ultimately selected as liver tissue-specific markers. In Table 4 and Table 5 below, the information and sequences of cg12137206 cg03792768 markers were described.

**[Table 4]**

| **Probe_ID** | **CGRC_ID** | **Gene_ID** | **chr** | **start** | **end** | **CGI** | **CGI_loci** |
|---|---|---|---|---|---|---|---|
| cg121372 06 | CGRC_LT 0.1 | GPAM | chr10 | 113943397 | 113943398 | chr10:11394328 3-113943657 | pCGI |
| cg037927 68 | CGRC_LT O.2 | BDH1 | chr3 | 197281934 | 197281935 | chr3:197281605 -197283128 | pCGI |

**[Table 5]**

| **Probe ID** | **Sequence** |
|---|---|
| **cg12137206** | GGGGACACGACTGCCCCAGCAACTTGCAGGAGTCGCACCACCTCCA TGCACTTGTCCCGG[CG]CTCCCGGCCCGAGTAGCCTCCCGCAGCCCA CACCTGCCCTGGCAGTTCGCACCCTAGCAG (SEQ ID NO: 1) |
| **cg03792768** | TTCCCATTGGTTGAGACAGCACCGCCCAGCCAAAGCCCCCTTGTCCT CGCGCGGGTGCGC[CG]CCTGGACTCCCACCCTGGCCAGTCCCGGGC CCACCACCACTCTGGCATCCCCAGCCTGTC (SEQ ID NO: 2) |

| | |
|---|---|
| - In the table, [CG] means a target methylation site of each probe. | |

### Example 3: Validation of finally selected liver tissue-specific markers

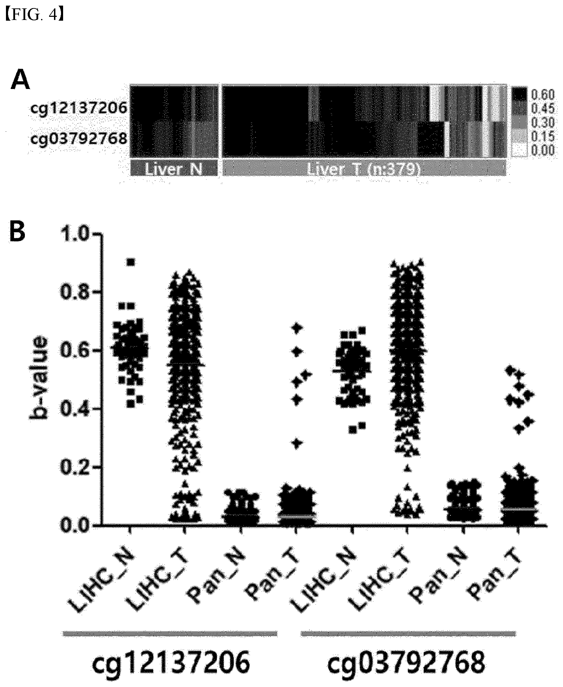

The methylation level of the finally selected cg12137206 and cg03792768 markers in the live tissue and other tissue was confirmed. As a result, as shown in A of FIG. 4, it could be seen that the two markers were methylated at a high level in both the normal liver tissue (Liver N) and liver cancer tissue (Liver T), and as shown in B of FIG. 4, it could be seen that the two markers had a low methylation level in all the other cancer tissues and other normal tissues.

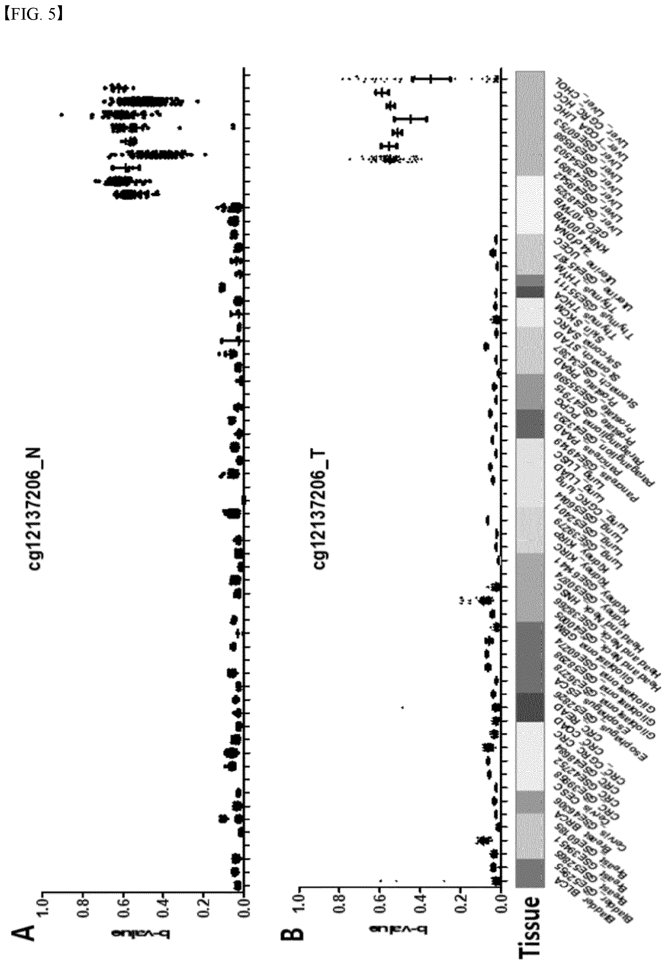

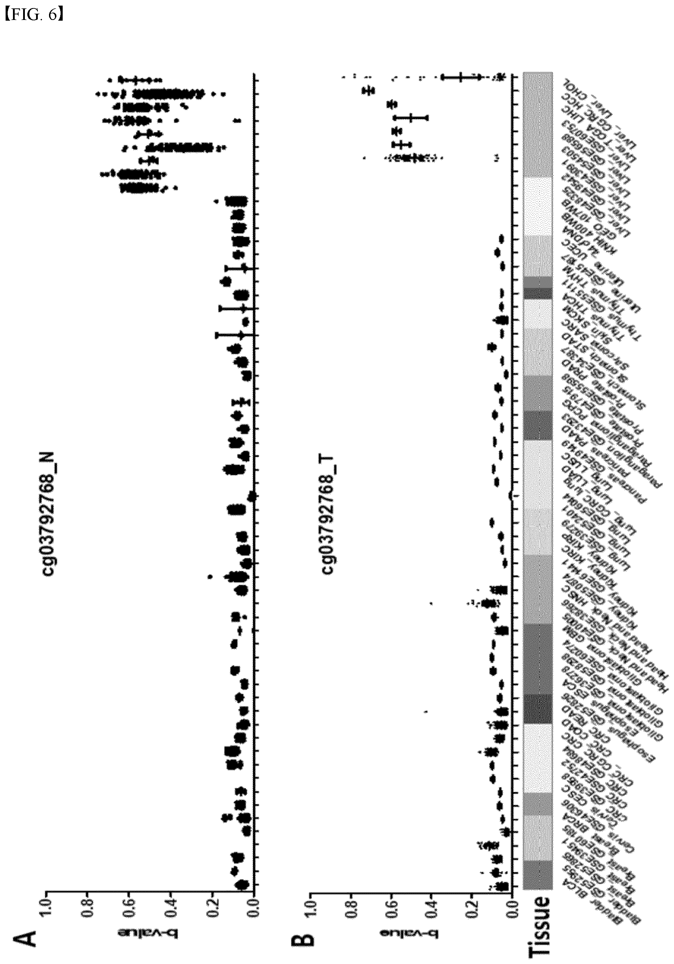

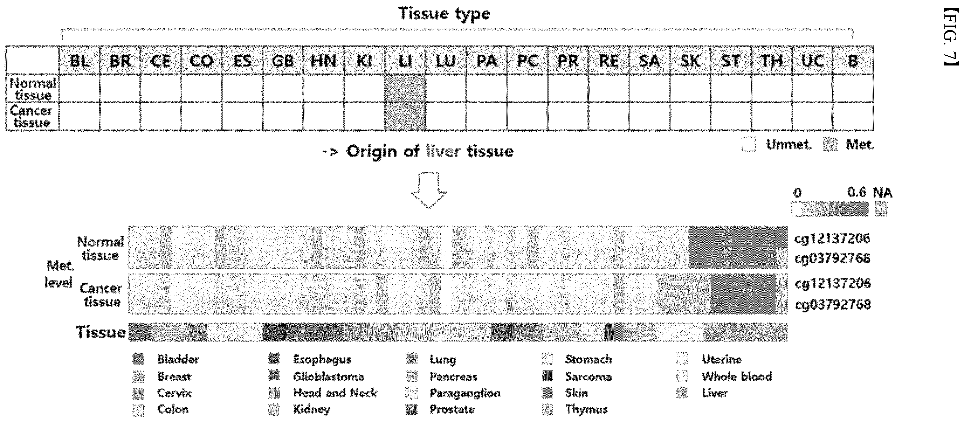

In FIG. 5 to FIG. 7, the methylation levels of cg12137206 and cg03792768 markers confirmed in the liver tissue and other tissues were shown, and in Tables 6 to 8, the result of cross validation of the markers was described.

**[Table 6]**

| **Tissue** | | **Sample number** | **Accuracy_N** | **Accuracy_T** |
|---|---|---|---|---|
| Blood (whole blood, B) | KNIH | 400 | 1 | NA |
| | GEO_WBset | 107 | 1 | NA |
| liver tumorigenesis | GSE48325 | 79 | 1 | NA |
| | GSE49542 | 59 | 1 | NA |
| Hepatocellular carcinoma (HCC) | GSE43091 | 54 | 1 | 0.94 |
| | GSE54503 | 132 | 1 | 0.9851 |
| | GSE56588 | 234 | 1 | 0.9872 |
| | GSE60753 | 66 | 0.9412 | 0.9375 |
| | TCGA_LIHC | 379 | 1 | 0.9921 |
| | CGRC_HCC | 307 | 1 | 0.9835 |
| | TCGA_CHOL | 45 | 1 | 0.5278 |
| Other tissue | CGRC_CRC | 709 | 1 | 1 |
| | CGRC_lung | 42 | NA | 1 |

**[Table 7]**

| **Tissue** | **TCGA** | **Sample number** | **Accuracy_N** | **Accuracy_T** |
|---|---|---|---|---|
| Bladder (BL) | BLCA | 434 | 1 | 1 |
| Breast (BR) | BRCA | 869 | 1 | 1 |
| Cervix (CE) | CESC | 312 | 1 | 1 |
| Colon (CO) | COAD | 335 | 1 | 1 |
| Rectum (RE) | READ | 106 | 1 | 1 |
| Esophagus (ES) | ESCA | 202 | 1 | 1 |
| Glioblastoma (GB) | GBM | 154 | 1 | 1 |
| Head and neck (HN) | HNSC | 580 | 1 | 1 |
| Kidney (KI) | KIRC | 480 | 1 | 1 |
| Kidney (KI) | KIRP | 321 | 1 | 1 |
| Lung (LU) | LUAD | 492 | 1 | 1 |
| Lung (LU) | LUSC | 412 | 1 | 1 |
| Pancreas (PA) | PAAD | 195 | 1 | 1 |
| Paraganglioma (PC) | PCPG | 187 | 1 | 1 |
| Prostate (PR) | PRAD | 549 | 1 | 1 |
| Stomach (ST) | STAD | 397 | 1 | 1 |
| Sarcoma (SA) | SARC | 269 | 1 | 1 |
| Skin (SK) | SKCM | 475 | 1 | 1 |
| Thymus (TH) | THCA | 574 | 1 | 1 |
| Thymus (TH) | THYM | 126 | 1 | 1 |
| Uterine (UC) | UCEC | 466 | 1 | 1 |

**[Table 8]**

| **Tissue** | **GEO** # | **Sample number** | **Accuracy** |
|---|---|---|---|
| Bladder (BL) | GSE52955 | 30 | 1 |
| Breast (BR) | GSE52865 | 57 | 1 |
| | GSE39451 | 20 | 1 |
| | GSE60185 | 285 | 1 |
| Cervix (CE) | GSE46306 | 44 | 1 |
| Colon (CO) | GSE39958 | 45 | 1 |
| | GSE42752 | 63 | 1 |
| | GSE48684 | 147 | 1 |
| Rectum (ES) | GSE52826 | 12 | 1 |
| Glioblastoma (GB) | GSE36278 | 142 | 0.9787 |
| | GSE58298 | 40 | 1 |
| | GSE60274 | 77 | 0.987 |
| Head and neck (HN) | GSE40005 | 24 | 1 |
| | GSE38266 | 42 | 0.9286 |
| Kidney (KI) | GSE50874 | 85 | 1 |
| | GSE61441 | 92 | 1 |
| Lung (LU) | GSE39279 | 444 | 1 |
| | GSE52401 | 244 | 1 |
| | GSE56044 | 136 | 1 |
| Pancreas (PA) | GSE49149 | 196 | 1 |
| Paraganglioma (PC) | GSE43293 | 24 | 1 |
| Prostate (PR) | GSE47915 | 8 | 1 |
| | GSE55598 | 48 | 1 |
| Stomach (ST) | GSE34387 | 76 | 0.9867 |
| Thymus (TH) | GSE55111 | 11 | 1 |
| Uterine (UC) | GSE45187 | 9 | 1 |

From the results so far, it can be seen that cg12137206 and cg03792768 markers have a high methylation level in liver tissue and have a low methylation level in other tissue, and thus, the two markers can be used as a liver tissue-specific marker.

## Claims

1. A method for determining whether a biological sample originates from liver tissue comprising;
(a) separating DNA from a biological sample isolated from a subject; and
(b) measuring methylation levels of CpG site of the sequences represented by SEQ ID NO: 1 and SEQ ID NO: 2 in the separated DNA.

2. The method for determining whether a biological sample originates from liver tissue according to claim 1, wherein the biological sample is selected from the group consisting of tissue, tissue fragments, cells, cell fragments, blood, plasma, body fluids, feces and urine isolated from the subject.

3. The method for determining whether a biological sample originates from liver tissue according to claim 1,
wherein the (b) is performed by a method selected from the group consisting of PCR, methylation specific PCR, real time methylation specific PCR, MethyLight PCR, MehtyLight digital PCR, EpiTYPER, PCR using methylated DNA specific binding protein, quantitative PCR, DNA chip, molecular beacon, MS-HRM (Methylation-sensitive high resolution melting), asymmetric PCR, asymmetric PCR MS-HRMA (asymmetric PCR Methylation-sensitive high resolution melting analysis), Recombinase Polymerase Amplification, LAMP (Loop-Mediated Isothermal Amplification), Eclipse probe, next generation sequencing panel (NGS panel), pyrosequencing and bisulfide sequencing.

4. A method for detecting liver tissue-derived DNA in a biological sample comprising
(a) separating DNA from a biological sample isolated from a subject; and
(b) measuring methylation levels of CpG site of the sequences represented by SEQ ID NO: 1 and SEQ ID NO: 2 in the separated DNA.

5. A composition for determining whether a biological sample originates from liver tissue comprising agents capable of measuring methylation levels of the sequences represented by SEQ ID NO: 1 and SEQ ID NO: 2.

6. The composition for determining whether a biological sample originates from liver tissue according to claim 5, wherein the agents capable of measuring methylation levels are primers, probes or antisense nucleic acids binding to CpG sites of the sequences represented by SEQ ID NO: 1 and SEQ ID NO: 2.
